# EUROPÄISCHE PATENTANMELDUNG

(11) **EP 1 772 162 A1**
(43) Veröffentlichungstag der Anmeldung: **11.04.2007**
(21) Anmeldenummer: 05021982.3
(22) Anmeldetag: 08.10.2005
(51) Int. Cl.: A61M 5/168, A61M 5/142, A61M 5/172

(54) **Infusionssystem zum Verabreichen eines flüssigen Medikaments**

(71) Anmelder: Roche Diagnostics GmbH, 68305 Mannheim (DE); F. Hoffmann-La Roche AG, 4070 Basel (CH)
(72) Erfinder: Haar, Hans-Peter, 69168 Wiesloch (DE)
(74) Vertreter: Jany, Peter

(57) **Zusammenfassung**

Die Anmeldung betrifft ein Infusionssystem Infusionssystern (1) zum Verabreichen eines flüssigen Medikaments, insbesondere Insulin, umfassend eine Pumpe (2), mit der durch einen Infusionsschlauch (3) das Medikament in den Körper eines Patienten gepumpt werden kann, und eine Steuereinheit (4) zum Steuern der Pumpe (2). Das Infusionssystem (1) umfaßt einen Drucksensor (3) zum Überwachen des in dem Infusionsschlauch (3) herrschenden Flüssigkeitsdrucks.

## Beschreibung

Die Erfindung betrifft ein Infusionssystem zum Verabreichen eines flüssigen Medikaments insbesondere Insulin, umfassend eine Pumpe, mit der durch einen Infusionsschlauch das Medikament in den Körper eines Patienten gepumpt werden kann, und eine Steuereinheit zum Steuern der Pumpe. Ein derartiges Infusionssystem ist beispielsweise aus der US 2005/0137573 A bekannt.

Viele Diabetiker sind zur Regulierung ihres Blutglukosespiegels auf externe Insulingaben angewiesen. Üblicherweise erfolgen diese Insulingaben mehrmals täglich in Form von Injektionen. Obwohl aus medizinischer Sicht kontinuierliche Insulingaben vorteilhaft sind; haben sich Infusionssysteme zum kontinuierlichen Verabreichen von Insulin bisher aus verschiedenen Gründen nicht durchsetzen können. Einerseits ist der Tragekomfort bekannter Infusionssysteme unzureichend, andererseits muß ein Benutzer in regelmäßigen Zeitabständen die korrekte Arbeitsweise seines Infusionssystem überprüfen, um sicherzustellen, daß tatsächlich die gewünschte Insulindosis verabreicht wird.

Aufgabe der Erfindung ist es, ein verbessertes Infusionssystem mit einer erhöhten Betriebssicherheit zu schaffen.

Diese Aufgabe wird erfindungsgemäß durch einen Drucksensor zum Überwachen des in dem Infusionsschlauch herrschenden Flüssigkeitsdrucks gelöst.

Durch Auswertung des in dem Infusionsschlauch herrschenden Flüssigkeitsdrucks kann festgestellt werden, ob das Medikament mit der angestrebten Infusionsrate in den Körper des Patienten gepumpt wird. Beispielsweise kann ein Blutgerinsel an der Auslaßöffnung des Infusionsschlauchs dazu führen, daß weniger als die eingestellte Medikamentenmenge in den Körper eingeleitet wird oder das Einleiten des Medikaments wegen einer Verstopfung der Auslaßöffnung sogar ganz zum Erliegen kommt. Dies führt zu einem Anstieg des Flüssigkeitsdrucks in dem Infusionsschlauch, der mit dem Drucksensor festgestellt werden kann. Umgekehrt kann ein Absinken des Flüssigkeitsdrucks in dem Infusionsschlauch darauf hindeuten, daß das Medikament aus dem Infusionsschlauch austritt ohne in den Körper des Patienten zu gelangen, beispielsweise wegen eines Lecks oder weil sich ein Katheter gelöst hat.

Wird anhand der mittels des Drucksensors ermittelten Druckdaten festgestellt, daß der Flüssigkeitsdruck von einem Sollwert um mehr als einen vorgegebenen Schwellenwert abweicht, kann dies einem Benutzer mittels einer Signaleinrichtung, beispielsweise durch Vibration oder ein akustisches Signal, angezeigt werden. Ein Benutzer kann auf diese Weise auf eine Fehlfunktion des Infusionssystems aufmerksam gemacht werden, so daß diese behoben werden kann.

Bevorzugt erzeugt die Steuereinheit in Abhängigkeit von Druckdaten, die von dem Drucksensor ermittelt wurden, Steuersignale zur Steuerung der Pumpe. Bei einer Verstopfung des Infusionsschlauchs besteht nämlich die Gefahr, daß sich über einen längere Zeitraum von mehreren Stunden in dem Infusionsschlauch bei stetig steigendem Flüssigkeitsdruck eine größere Medikamentenmenge ansammelt. Löst sich die Verstopfung schließlich unter Einwirkung des erhöhten Flüssigkeitsdrucks, so wird innerhalb kürzester Zeit in den Körper die gesamte angestaute Medikamentenmenge eingeleitet. Handelt es sich bei dem Medikament um Insulin, so kann dies zu einem gefährlichen Absinken des Blutglukosegehalts führen. Eine derartige Fehlfunktion des Infusionssystems kann verhindert werden, indem die Steuereinheit so ausgebildet ist, daß die Pumpe abgeschaltet wird, wenn anhand von mittels des Drucksensors ermittelten Druckdaten festgestellt wird, daß der Flüssigkeitsdruck einen Schwellenwert übersteigt. Bei weniger gravierenden Änderungen des Flüssigkeitsdrucks kann durch geeignete Steuersignale der Steuereinheit die Pumprate nach Bedarf erhöht oder gesenkt werden.

Für einen möglichst hohen Tragekomfort ist es günstig, eine externe Steuereinheit zu verwenden. Auf diese Weise lassen sich die für die Pumpe erforderlichen Teile auf ein Minimum beschränken, so daß sich eine besonders kompakte und leichte Pumpe realisieren läßt. Die Betriebssicherheit eines derartigen Infusionssystems läßt sich dadurch erhöhen, daß der Infusionsschlauch Träger einer Datenleitung zur Kommunikation des Steuergeräts mit der Pumpe und/oder dem Drucksensor ist. Auf diese Weise kann man die bei einer drahtlosen Kommunikation bestehende Gefahr ausschließen, daß durch das Steuergerät eines ersten Patienten die Pumpe eines in der Nähe befindlichen zweiten Patienten fehlgesteuert wird.

Ein wichtiger Aspekt der Erfindung betrifft deshalb ein Infusionssystem zum Verabreichen eines flüssigen Medikaments, insbesondere Insulin, umfassend eine Pumpe, mit der durch einen Infusionsschlauch das Medikament in den Körper eines Patienten gepumpt werden kann, und eine Steuereinheit zum Steuern der Pumpe, dadurch gekennzeichnet, daß der Infusionsschlauch Träger einer Datenleitung zur Kommunikation der Steuereinheit mit der Pumpe und/oder dem Drucksensor ist. Selbstverständlich kann ein derartiges Infusionssystem auch mit einem Drucksensor zum Überwachen des in dem Infusionsschlauch herrschenden Flüssigkeitsdrucks ausgerüstet werden, um die Betriebssicherheit noch weiter zu erhöhen.

Die im vorhergehenden genannte Aufgabe der Erfindung wird ferner durch einen Infusionsschlauch für ein erfindungsgemäßes Infusionssystem gelöst, der eine Metallschicht aufweist. Diese Metallschicht kann als Sensorfläche eines kapazitiv arbeitenden Drucksensors dienen, mit dem ein Aufblähen des Infusionsschlauchs erfaßt wird. Die Metallschicht kann ferner als elektrische Datenleitung verwendet werden, über die das Steuergerät, die Pumpe und/oder der Drucksensor Daten austauschen können.

Weitere Einzelheiten und Vorteile der Erfindung werden anhand eines Ausführungsbeispiels unter Bezugnahme auf die beigefügten Zeichnungen erläutert. Die im folgenden beschriebenen Merkmale können einzeln oder in Kombination verwendet werden, um bevorzugte Ausgestaltungen der Erfindung zu schaffen. Es zeigen:
- Fig. 1: ein erfindungsgemäßes Infusionssystem; und
- Fig. 2: eine schematische Darstellung der Funktionsweise des Drucksensors des in Fig. 1 dargestellten Infusionssystems.

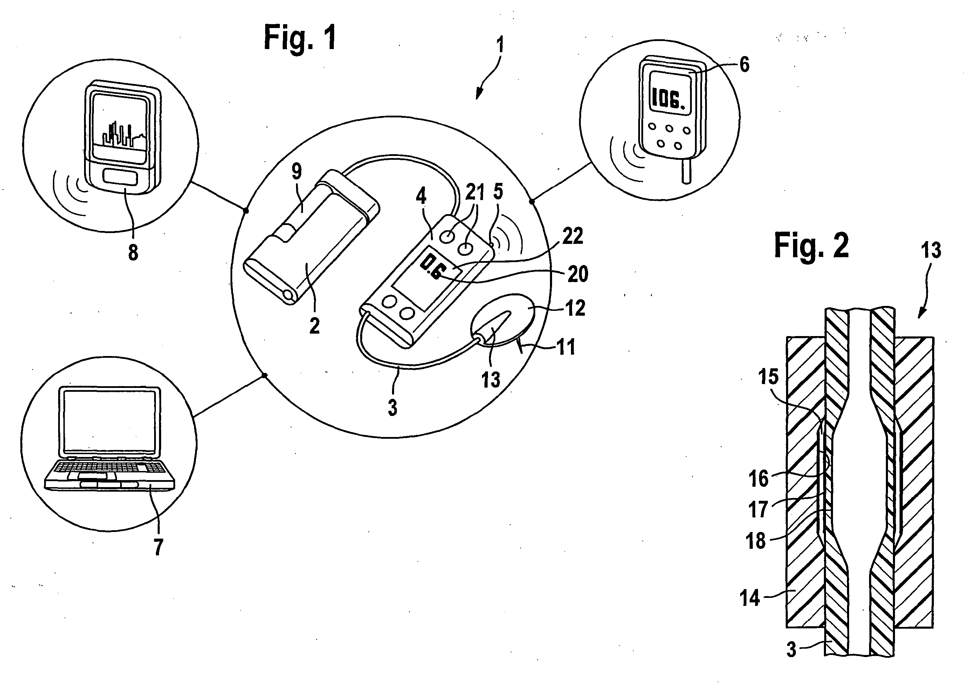

Das in Fig. 1 dargestellte Infusionssystem 1 zum Verabreichen eines flüssigen Medikaments, insbesondere Insulin, umfaßt eine Pumpe 2, mit der durch einen Infusionsschlauch 3 das Medikament in den Körper eines Patienten gepumpt werden kann, und eine externe Steuereinheit 4 zum Steuern der Pumpe 2. Die externe Steuereinheit 4 weist eine Infrarot-Schnittstelle 5 mit einem Infrarot-Sender und einem Infrarot-Empfänger zur Kommunikation mit externen Geräten auf. Als Beispiele externer Geräte sind ein Blutglukosemeßgerät 6, ein PC 7 und ein PDA (Personal Data Assistent) 8 dargestellt.

In die Pumpe 2 kann eine Medikamentenkartusche 9 eingesetzt werden, die das zu verabreichende Medikament enthält. Die Pumpe 2 und das Steuergerät 4 haben jeweils eine interne Energieversorgung in Form von handelsüblichen Batterien, bevorzugt wiederaufladbaren Batterien.

Der Infusionsschlauch 3 mündet in einen Katheter 11, der bestimmungsgemäß in den Körper eines Patienten hineinragt. Der Katheter 11 wird in der üblichen Weise mit einem Klebekissen 12 fixiert, das auf die Haut des Patienten geklebt wird. Das Klebekissen 12 trägt ferner einen Drucksensor 13 zum Überwachen des in dem Infusionsschlauch 3 herrschenden Flüssigkeitsdrucks.

Der Aufbau des Drucksensors 13 ist schematisch in Fig. 2 dargestellt. Der Drucksensor 13 umfaßt ein Gehäuse 14, durch das der Infusionsschlauch 3 hindurchgeführt ist. In dem Gehäuse 14 befindet sich eine Kammer 15, deren Innenwand eine erste Sensorfläche 16 bildet, die einer zweiten Sensorfläche 17, die der in der Kammer befindliche Schlauchabschnitts 18 des Infusionsschlauchs 3 trägt, gegenüberliegt. Bei dem dargestellten Ausführungsbeispiel umgibt die erste Sensorfläche 16 zylinderförmig die von einer Außenfläche des Schlauchabschnitts 18 gebildete zweite Sensorfläche 17. Die beiden Sensorflächen 16, 17 bilden einen Kondensator, dessen Kapazität sich beim Aufblähen des Schlauchabschnitts 18 ändert. Die Sensorflächen 16 und 17 sind Metallschichten. Sowohl das Sensorgehäuse 14 als auch der Infusionsschlauch 3 sind im übrigen aus Kunststoff gefertigt.

Auf diese Weise ist der Drucksensor 13 derart ausgebildet, daß der Flüssigkeitsdruck dadurch überwacht wird, daß ein von dem Flüssigkeitsdruck bewirktes Aufblähen des Schlauchabschnitts 18 des Infusionsschlauches 3 erfaßt wird. Ein Aufblähen des Schlauchabschnitts 18 führt zu einer Änderung der Kapazität des von den Sensorflächen 16, 17 gebildeten Kondensators. Der Kondensator ist Teil eines elektrischen Schwingkreises (nicht dargestellt), dessen Resonanzfrequenz von der Kapazität des Kondensators 16, 17 abhängt. Zur Bestimmung des Flüssigkeitsdrucks wird die Frequenz des Schwingkreises gemessen und daraus die Kapazität des Kondensators 16, 17 oder mittels einer geeigneten Eichkurve unmittelbar der damit verknüpfte Flüssigkeitsdruck ermittelt.

Der Schlauchabschnitt 18, dessen Aufblähen von dem Drucksensor 13 erfaßt wird, weist eine geringere Wandstärke als benachbarte Schlauchabschnitte auf. Auf diese Weise läßt sich die Meßsensivität des Drucksensors 13 erhöhen. Bevorzugt hat der Infusionsschlauch 3 einen Außendurchmesser von 150 bis 300 µm, bevorzugt 180 bis 220 µm, und eine Wandstärke von 50 bis 200 µm, bevorzugt 70 bis 150 µm, besonders bevorzugt 80 bis 120 µm. In dem Schlauchabschnitt 18, dessen Aufblähen von dem Drucksensor 13 erfaßt wird, beträgt die Wandstärke weniger als 60%, bevorzugt nur 20% bis 50%, besonders bevorzugt 30% bis 40%, der Wandstärke benachbarter Schlauchabschnitte.

Von dem Drucksensor 13 ermittelte Druckdaten werden an die Steuereinheit 4 weitergeleitet und dort ausgewertet. Die Steuereinheit 4 erzeugt in Abhängigkeit von Druckdaten, die von dem Drucksensor 13 ermittelt wurden, Steuersignale zur Steuerung der Pumpe 2. Die Steuereinheit 4 ist dabei derart ausgebildet, daß die Pumpe 2 abgeschaltet wird, wenn anhand von mittels des Drucksensors 13 ermittelten Druckdaten festgestellt wird, daß der Flüssigkeitsdruck einen Schwellenwert übersteigt.

Die externe Steuereinheit 4 umfaßt ferner eine Signal- und Anzeigeeinrichtung 20, mit der ein Benutzer durch geeignete Signale darauf aufmerksam gemacht werden kann, daß der Flüssigkeitsdruck um mehr als einen vorgegebenen Schwellenwert von einem Sollwert abweicht. Eine derartige Abweichung deutet auf eine Fehlfunktion des Infusionsystems 1 hin und wird dem Benutzer mittels der Signal- und Anzeigeeinrichtung 20 durch ein akkustisches Signal und/oder durch Vibration angezeigt.

Die externe Steuereinheit 4 weist ferner Bedienelemente 21 in Form von Tasten auf, mit denen in Verbindung mit einem Display 22 der Signal- und Anzeigeeinrichtung 20 das Infusionssystem 1 bedient und beispielsweise eine gewünschte Infusionsrate eingestellt werden kann.

Bei dem dargestellten Infusionssystem 1 ist der Infusionsschlauch 3 Träger einer Datenleitung zur Kommunikation des Steuergeräts 4 mit der Pumpe 2 und dem Drucksensor 13. Die Datenleitung dient dabei auch zur Energieversorgung des Drucksensors 13. Bevorzugt handelt es sich bei der Datenleitung um eine Infrarot-Datenleitung. Beispielsweise kann der Infusionsschlauch 3 Träger einer optischen Faser sein, die in den Schlauch 3 eingebettet ist oder auf ihm angeordnet ist. Das Steuergerät 4 und die Pumpe 2 enthalten zu diesem Zweck jeweils einen Infrarot-Laser, bevorzugt einen VCSEL-Laser (Vertical Cavity Surface Emitting Laser) zum Erzeugen von Datenpulsen und eine wellenlängenangepaßte Fotodiode zum Empfangen der Datenpulse.

Alternativ kann die Datenleitung auch eine elektrische Datenleitung sein. Durch Einbetten eines elektrischen Leiters, bevorzugt einer Metallschicht, in den Infusionsschlauch 3 läßt sich eine elektrische Datenleitung mit geringem Aufwand realisieren. Bevorzugt ist die Datenleitung kapazitiv an die Pumpe 2 angekoppelt. Eine kapazitive Ankopplung hat den Vorteil, daß in dem hermetisch verschlossenen Gehäuse der Pumpe 2 keine Öffnung mit einer dazugehörenden Dichtstelle zum Durchführen der Datenleitung geschaffen werden muß. Eine kapazitive Ankopplung läßt sich am einfachsten durch einen metallisierten Infusionsschlauch 3 und eine Elektrodenfläche an einer Innenfläche eines Gehäuses der Pumpe 2 realisieren. Die Elektrodenfläche der Pumpe 2 bildet dann mit dem metallisierten Infusionsschlauch 3 einen Kondensator, über den Daten übertragen werden können. Die Vorteile einer kapazitiven Ankopplung der Datenleitung lassen sich, auch für die Steuereinheit 4 und den Drucksensor 13 nutzen.

Bevorzugt ist in das Klebekissen 12 und den Katheter 11 ein Glukosesensor integriert, mit dem die Glukosekonzentration im Blut oder interstitieller Flüssigkeit des Patienten kontinuierlich gemessen werden kann. Von dem Glukosesensor ermittelte Meßdaten werden über die von dem Infusionsschlauch 3 gebildete Datenleitung an die Steuereinheit 4 übertragen und dort ausgewertet. Die Meßwerte können dann mit dem Display 22 angezeigt werden. Bevorzugt erzeugt die Steuereinheit 4 in Abhängigkeit von den Meßdaten, die von dem Glukosesensor ermittelt wurden, Steuersignale zur Steuerung der Pumpe 2, so daß die Infusionsrate von Insulin an den aktuellen Bedarf des Patienten angepaßt werden kann.

Die Arbeitsweise der Pumpe 2 beruht bevorzugt auf einem Kolbenelement (nicht dargestellt), das von einem Pumpenantrieb in die Kartusche 9 hineinbewegt wird, so daß in der Kartusche 9 enthaltene Flüssigkeit in den Infusionsschlauch 3 gepreßt wird. Bevorzugt ist das Kolbenelement biegsam, beispielsweise eine Schraubenfeder, so daß sich die Baulänge der Pumpe 2 auf ein Mindestmaß reduzieren läßt. Für einen Feuchtigkeitsschutz der Pumpe 2 ist es günstig, das Kolbenelement mit einer Gummitülle (nicht dargestellt) hermetisch zu kapseln.

Das externe Steuergerät 4 ist zur Erhöhung des Benutzerkomforts entlang des Infusionsschlauchs in eine beliebige Stellung verschieblich. Dies wird dadurch erreicht, daß der Infusionsschlauch 3 durch einen in der Steuereinheit 4 enthaltenen Kanal hindurchgeführt ist. Der Benutzerkomfort läßt sich ferner dadurch erhöhen, daß das Infusionssystem 1 mehrere, bevorzugt zwei, Pumpen 2 enthält, die abwechselnd verwendet werden können. Auf diese Weise kann jeweils die nicht verwendete Pumpe in eine Basisstation zum Aufladen ihrer wiederaufladbaren Batterien gestellt werden.

## Patentansprüche

1. Infusionssystem zum Verabreichen eines flüssigen Medikaments, insbesondere Insulin, umfassend
eine Pumpe (2), mit der durch einen Infusionsschlauch (3) das Medikament in den Körper eines Patienten gepumpt werden kann, und
eine Steuereinheit (4) zum Steuern der Pumpe (2),
**gekennzeichnet durch**
einen Drucksensor (3) zum Überwachen des in dem Infusionsschlauch (3) herrschenden Flüssigkeitsdrucks.

2. Infusionssystem nach Anspruch 1, **dadurch gekennzeichnet, daß** die Steuereinheit (4) in Abhängigkeit von Druckdaten, die von dem Drucksensor (13) ermittelt wurden, Steuersignale zur Steuerung der Pumpe (2) erzeugt.

3. Infusionssystem nach einem der vorhergehenden Ansprüche, **dadurch gekennzeichnet, daß** die Steuereinheit (4) derart ausgebildet ist, daß die Pumpe (2) abgeschaltet wird, wenn anhand von mittels des Drucksensors (13) ermittelten Druckdaten festgestellt wird, daß der Flüssigkeitsdruck einen Schwellenwert übersteigt.

4. Infusionssystem nach einem der vorhergehenden Ansprüche, **dadurch gekennzeichnet, daß** der Drucksensor (13) derart ausgebildet ist, daß der Flüssigkeitsdruck **dadurch** überwacht wird, daß ein von dem Flüssigkeitsdruck bewirktes Aufblähen eines Abschnitts (18) des Infusionssschlauchs (3) erfaßt wird.

5. Infusionssystem nach Anspruch 4, **dadurch gekennzeichnet, daß** der Schlauchabschnitt (18), dessen Aufblähen von dem Drucksensor (13) erfaßt wird, eine geringere Wandstärke als benachbarte Schlauchabschnitte aufweist.

6. Infusionssystem nach Anspruch 4 oder 5, **dadurch gekennzeichnet, daß** der Schlauchabschnitt (18), dessen Aufblähen von dem Drucksensor (13) erfaßt wird, eine Sensorfläche (17) trägt, die mit einer gegenüberliegenden Sensorfläche (16) des Drucksensors (13) einen Kondensator bildet, dessen Kapazität sich beim Aufblähen des Schlauchabschnitts (18) ändert.

7. Infusionssystem zum Verabreichen eines flüssigen Medikaments, insbesondere Insulin, umfassend eine Pumpe (2), mit der durch einen Infusionsschlauch (3) das Medikament in den Körper eines Patienten gepumpt werden kann, und eine Steuereinheit (4) zum Steuern der Pumpe (2), **dadurch gekennzeichnet, daß** der Infusionsschlauch (3) Träger einer Datenleitung zur Kommunikation der Steuereinheit (4) mit der Pumpe (2) und/oder dem Drucksensor (13) ist.

8. Infusionssystem nach Anspruch 7, **dadurch gekennzeichnet, daß** die Datenleitung eine Infrarot-Datenleitung ist.

9. Infusionssystem nach Anspruch 8, **dadurch gekennzeichnet, daß** die Datenleitung eine elektrische Datenleitung ist, wobei die Datenleitung kapazitiv an die Steuereinheit (4), die Pumpe (2) und/oder den Drucksensor (13) angekoppelt ist.

10. Infusionssystem nach einem der vorhergehenden Ansprüche, **dadurch gekennzeichnet, daß** die Steuereinheit (4) eine Schnittstelle, vorzugsweise eine drahtlose Schnittstelle, zur Kommunikation mit anderen Geräten (6,7,8), beispielsweise einem Blutglukosemeßgerät (6), aufweist.

11. Infusionsschlauch für ein Infusionssystem nach einem der vorhergehenden Ansprüche, **dadurch gekennzeichnet, daß** der Infusionsschlauch (3) eine Metallschicht (16) trägt.

12. Infusionsschlauch nach Anspruch 11, **gekennzeichnet durch** einen Schlauchabschnitt (18) mit einer Wandstärke, die weniger als 60% der Wandstärke benachbarter Wandabschnitte beträgt.

13. Infusionsschlauch nach Anspruch 11 oder 12, **dadurch gekennzeichnet, daß** der Infusionsschlauch (3) eine Datenleitung trägt.
